# EUROPEAN PATENT SPECIFICATION

(11) **EP 0 889 735 B1**
(45) Date of publication and mention of the grant of the patent: **13.04.2011**
(21) Application number: 97905858.3
(22) Date of filing: 07.02.1997
(51) Int. Cl.: A61K 39/385, C07K 14/595, C07K 16/26

(54) **IMMUNOLOGICAL METHODS FOR THE TREATMENT OF GASTROINTESTINAL CANCER**
IMMUNOLOGISCHE METHODEN ZUR BEHANDLUNG VON GASTROINTESTINALEM KREBS
PROCEDES IMMUNOLOGIQUES APPLIQUES DANS LE TRAITEMENT DU CANCER DU TUBE DIGESTIF

(30) Priority: 08.02.1996 US 11411 P
(43) Date of publication of application: 13.01.1999
(73) Proprietor: Cancer Advances, Inc.,, Durham NC (US)
(72) Inventor: GEVAS, Philip, C., Honolulu, HI 96825 (US); KARR, Stephen, L., Davis, CA 95616 (US); GRIMES, Stephen, Davis, CA 95616 (US); MICHAELI, Dov, Larkspur, CA 94939 (US); WATSON, Susan, A., Nottingham NG2 6RB (GB)
(74) Representative: Baldock, Sharon Claire
(86) International application number: PCT/US1997/002029
(87) International publication number: WO 1997/028821

(56) References cited:
- EP-A- 0 380 230
- US-A- 5 023 077
- US-A- 5 468 494
- INT. J. CANCER (1995), 61(2), 233-40 CODEN: IJCNAW;ISSN: 0020-7136, 1995, XP000654518 WATSON, SUSAN A. ET AL: "Anti - gastrin antibodies raised by gastrimmune inhibit growth of the human colorectal tumor AP5"
- EXP. CELL RES. (1990), 186(1), 15-21 CODEN: ECREAL;ISSN: 0014-4827, 1990, XP000654524 HOOSEIN, NASEEMA M. ET AL: "Evidence for autocrine growth stimulation of cultured colon tumor cells by a gastrin /cholecystokinin-like peptide"

## Description

### BACKGROUND OF THE INVENTION

The mature gastrin hormone occurs in two molecular forms which are named with respect to the number of amino acids in the peptide, i.e., tetratriacontagastrin (G34) and heptadecagastrin (G17). In gastrin producing cells, these gastrin hormones are posttranslationally processed from a common precursor molecule termed "preprogastrin", which contains a signal peptide. The signal peptide "pre" is removed in the endoplasmic reticulum of the cell, resulting in the "progastrin" peptide, which is in turn further processed in the cell to yield the mature gastrins G34 and G17, before they are secreted into the bloodstream (Dickinson 1991). (The full citations for the references cited herein are provided in the Reference Section preceding the Claims). The mature forms of G34 and G17 are both amidated (NH2) at their carboxy terminal end. It has been elucidated that there are multiple forms of G17 resulting from differential processing of the precursor molecule, each of which may have different biological activities (Dickinson 1995 and Ciccotosto et al. 1995). The hormone gastrin is now a well recognized growth factor for human colorectal adenocarcinomas (see Watson et al. 1993 for a review). Elevated plasma levels of total gastrin occurs in patients with colorectal cancers, and in particular, increased amounts of the hormone precursor, progastrin, have been detected in many colorectal tumors using gastrin antisera (Ciccotosto et al. 1995).

Generally, in tumors such as those present in gastrin-dependent colon cancer, the cancer cells lose the ability to process prohormones to completion due to defects in the regulatory pathways of hormone secretion. This leads to the production and secretion of different molecular forms of the hormone. Colon carcinoma cells do not efficiently process progastrin and thus, produce mostly incomplete or aberrant gastrins, which results in less conversion of precursor gastrin to the mature peptides (Dickinson 1993 and Rehfeld et al. 1993). The increased gastrin level in colorectal tumors is, in part, attributed to the aberrant expression of the gastrin gene in the colorectal tumor cells (Hoosein et al. 1990, Baldwin et al. 1992 and Finley et al. 1993). Gastrin-like peptides have been identified in such cells (Hoosein et al. 1988, Watson et al. 1991 and Finley et al. 1993), and were confirmed to be precursor gastrin species (Van-Solinge et al. 1993 and Nemeth et al. 1993).

Serum-associated G17 has the potential to stimulate the growth of colorectal tumors in an endocrine manner mediated by CCKB/gastrin receptors (Watson et al. 1993). Gastrin-17 appears to be particularly implicated in stimulating the growth of human colorectal adenocarcinomas due to a possible increased affinity for gastrin/cholecystokinin (CCK) B receptors on the tumor cells, over other gastrin hormone, species (Rehfeld, J.F. 1972). The CCKB/gastrin receptors were found to be expressed in a high affinity form on 56.7% of human primary colorectal tumors (Upp et al. 1989). It has been postulated that a potential autocrine loop may also exist due to endogenous production of precursor gastrin peptides by such tumors (Van-Solinge et al. 1993 and Nemeth et al. 1993), as it has recently been shown that the precursor gastrin molecule, glycine-extended gastrin 17 (G17-Gly), stimulated the growth of a gastrointestinal tumor cell line. The trophic effects of G17-Gly on tumors has been shown to be mediated by a receptor other than the CCKB/gastrin receptor and an autocrine growth loop, possibly involving gastrin precursors, has been postulated to be involved in the proliferation of gastrointestinal tumors (Seva et al. 1994).

Available treatments for tumors stimulated or induced by gastrin, and for tumors that produce gastrin consists primarily of surgical resection of the cancerous tissue. This approach is frequently unsuccessful; in many instances, the tumors cannot be located or are present in anatomic sites that are inoperable. In most instances, These tumors do not respond well to radiation or chemotherapy regimens, and new treatments are needed to supplement present procedures.

A number of high affinity CCKB/gastrin receptor antagonists have been described, such as L-365,260 (Bock et al. 1989) and CI-988 (Hughes et al. 1990), which have been shown to effectively neutralize the effects of exogenous gastrin on gastrointestinal tumor growth both in vitro and in vivo (watson at al. and Romani et al. 1994). However, the antagonists lack specificity as they block the actions of all the potential ligands of the receptor, such as gastrin-34 (G34) and CCK. Moreover, the cellular receptors which recognize and bind the gastrin precursor, G17-Gly, do not bind all the inhibitors tested (Seva et al. 1994). Thus, if a distinct receptor is involved in the autoorine growth cascade, then the gastrin antagonists may be usable to block this mechanism of tumor growth promotion.

A therapeutic method of selectively inmunologically neutralizing the biological activity of the gastrin hormone would provide an effective means of controlling or preventing the pathologic changes resulting from excessive gastrin hormone production.

av. U.S. Patents Nos. 5,023,077 and 5,468,494 disclose immunogenic compositions useful for controlling G17 and G34 levels in a patient by generating anti-gastrin antibodies, and the use of such compositions for the treatment of gastric and duodenal ulcers and gastrin-induced cancers.

The immunogenic composition of US Patent 5,023,077 for active immunisation takes the form of a conjugated peptide comprising
(1) a fragment of gastrin-17 consisting of the first n N-terminal amino acids, where n is from 4 to 12, especially 4 to 8,
(2) an immunogenic carrier, preferably diphtheria toxoid, and optionally
(3) a spacer peptide, attached to the C-terminal end of the fragment, through which the gastrin-17 fragment is conjugated to the carrier. The spacer may be, for example, Lys Arg Pro Pro Pro Pro Lys, the lysines providing crosslinking.

The above US patent mentions antibodies to the above-defined fragment for passive immunisation.

European Patent EP-B 380,230 is similar.

US Patent 5,468,494 relates to two specific conjugated peptides of the above type, in which n is 9 and the peptide is cojugated to the carrier via a spacer Ser Ser Pro Pro Pro Pro Cys or Arg Pro Pro Pro Pro Cys.

Watson, S.A. et al., Anti-Gastrin antibodies raised by Gastrimmune inhibit growth of the human colorectal tumor AP5, Int. J. Cancer, 61: 233-240, 1995, describes the raising of antiserum in rabbits against Gastrimmune, an immunogen constructed of the N-terminal portion of human G17 conjugated to diptheria toxoid, and that this antiserum blocked basal in vitro growth of the human colorectal tumour cell line AP5.

### SUMMARY OF THE INVENTION

The present invention provides use of an anti-gastrin-17 immunogen in the manufacture of a formulation for treating mammalian gastrointestinal tumors dependent on glycine-extended gastrin-17, wherein:
the immunogen comprises an amino-terminal peptide of gastrin-17 conjugated to an immunogenic carrier by a spacer peptide, wherein the amino-terminal peptide of gastrin-17 comprises amino acids 1 to 9 of gastrin-17 and amino acids 1 to 9 are the sequence pGlu-Gly-Pro-Trp-Leu-Glu-Glu-Glu-Glu; and
the immunogen induces sufficient anti-gastrin 17 antibody titer to neutralize amidated gastrin-17 and glycine extended-gastrin-17.

Also provided is use of an anti-gastrin-17 antibody in the manufacture of a medicament for treating mammalian gastrointestinal tumors dependent on glycine-extended gastrin-17, wherein the antibody neutralizes amidated gastrin-17 and glycine exterrded-gastrin-17.

Additionally provided is an anti-gastria-17 immunogen for use in treating mammalian gastrointestinal tumors dependent on glycine-extended gastrin-17, wherein:
the immunogen comprises an amino-terminal peptide of gastrin-17 conjugated to an immunogenic carrier by a spacer peptide, wherein the amino-terminal peptide of gastrin-17 comprises amino acids 1 to 9 of gastrin-17, wherein amino acids 1 to 9 are the sequence pGlu-Gly-Pro-Trp-Leu-Glu-Glu-Glu-Glu; and
the immunogen induces sufficient anti-gastrin 17 antibody titer to neutralize amidated gastrin-17 and glycine extended-gastrin-17.

still further provided is an anti-gastrin-17 antibody for use in treating mammalian gastrointestinal tumors dependent on glycine-extended gastrin-17, wherein the antibody neutralizes amidated gastrin-17 and glycine extended gastrin-17.

Also described are immunological methods for the treatment of gastrin-dependent tumors which comprise the active or passive immunization of a patient with anti-G17 immunogen or antibodies against gastrin 17 hormone in order to control the patient's glycine-extended and amidated gastrin 17 levels. By inducing anti-gastrin 17 antibodies in a human patient, the hormone gastrin 17 and the prohormone progastrin G17-Gly are neutralized in vivo, so as to inhibit their physiological effects. In particular, the neutralization of G17 and the precursor G17-Gly prevents the binding of these peptides to their physiological receptors, thereby inhibiting the growth of the tumor cells.

The anti-G17 immunogens, comprise fragments of the N-terminal amino acids of G17 conjugated to an immunogenic carrier such as Diphtheria toxoid (DT), by a spacer peptide, and raise antibodies which bind both the amidated and glycine-extended forms of G17.

It is described that the method of immunization against amidated or glycine-extended G17 may preferably comprise active immunization, wherein a patient is immunized with an immunogen of the invention. The immunogen stimulates the production of antibodies against amidated and glycine extended G17 in the immunized patient, inducing sufficient antibody titers to neutralize and inhibit the physiological effects of amidated and glycine-extended G17 so as to limit the cancer-trophic hormone levels produced by the patient.

The physiological neutralization of progastrin G17-Gly hormone by the anti-G17 antibodies produced in the patient inhibits the of tumor cells dependent on progastrin G17-Gly as the growth stimulator or inducer. The treatment methods of the invention are particularly suited for the treatment of G17-Gly or amidated G17-responsive gastrointestinal cancers.

The immunogens of the invention comprise peptides composed of two functional regions: an immunomimic region and a spacer region. The function of the immunomimic region which immunologically crossreacts with G17 and G17-Gly, is to induce antibodies in the immunized animal that bind to the targeted G17 hormone, i.e. amidated and glycine extended G17, thereby inhibiting G17 function and arresting or slowing the growth of the G17-dependent tumor cell. The present immunogens induce a biologically effective immune response following administration of the immunogen in all immunized animals tested. The inmunominic peptide-spacer of this invention can be coupled to immunological carriers over a wide range of peptide to carrier substitution ratios and yield effective immunogen.

It is also described that the method of treatment may comprise passive immunization, in which antibodies against G17 are administered to the patient in a sufficient concentration to reduce the levels of circulating unbound G17 and G17-Gly. The reduced levels of free G17 and progastrin in the circulating blood of a patient as a result of anti-G17 antibody administration, results in an inhibition of the growth and size of the tumors. It is preferred that the anti-G17 antibodies for human therapy may be chimeric, humanized, or human monoclonal antibodies which may be produced by methods well known in the art.

### BRIEF DESCRIPTION OF THE DRAWINGS

**Figure 1** is a diagrammatical representation of glycine-extended G17, carboxy-amidated G17 and an anti-G17 immunogen (conjugated peptide) containing an amino terminal portion of G17.
**Figure 2** is a graphic representation of the displacement of [¹²⁵I]G17 from rabbit anti-human G17(1-9):DT (N-terseinal specific) antiserum by G17, glycine-extended G17 and G34.
**Figure 3** is a graphic representation of the displacement of [¹²⁵I]G17 from rabbit anti-human G17 (C-terainal specific) antiserum by G17, glycine-extended G17 and G34.
**Figure 4** depicts a bar graph on the effect of immunizations with the imnunogens used in the invention on the median cross-sectional areas of DHDK12 tumors. (Inter-quartile ranges for each median are present at the top of the respective columns).
**Figure 5** depicts a bar graph, on the effect of immunizations with the immunogens used in the invention on the final median weights of DHDK12 tumors (inter-quartile ranges for each median are present at the top of the respective columns).
**Figure 6** depicts the anti-rat G17 antibody levels of individual G17(1-9)- and DT-immunized rats (measured at a 1:100 dilution of sera).
   1 At tumor challenge (rat G17(1-9) treatment, 5
   2 At therapy termination (rat G17 (1-9) treatment, 7 immunizations)
   3 At tumor challenge (DT treatment, 5 immunizations)
   4 At therapy termination (DT treatment, 7 immunizations)
   5 Positive control (rat anti-rat G17:DT antiserum)
   6 Negative control (normal rat serum)

Antibody levels were measured by an ELISA capture assay in which anti-rat G17:DT antibodies were bound to rat G17-BSA coated on 96 well microtiter plates. Antibody binding was detected using an alkaline phosphatase based method with pNPP as substrate.

### DETAILED DESCRIPTION OF THE INVENTION

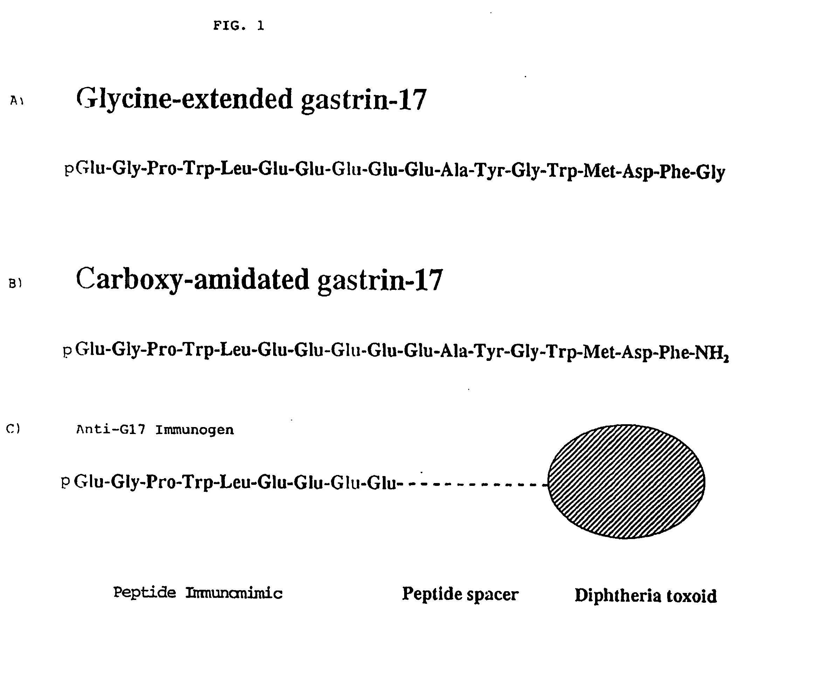

The methods described are directed to administering to a patient an anti-G17 immunogen which induces antibodies in the immunized patient which bind and neutralize amidated-G17 and glycine-extended G17 (see Figure 1).

Surprisingly, the immunogens and immunogenic compositions against G17 disclosed in co-assigned U.S. Patent Nos. 5,023,077 and 5,468,494, also produce antibodies in immunized animals which react with and neutralize amidated gastrin 17 and glycine-extended gastrin 17. Advantageously, therefore, these immunogens may be used in methods of treating cancer disease states which are trophic due to these precursor hormones.

U.S. Patent Nos. 5,023,077 and 5,468,494, disclose compositions containing anti-gastrin 17 immunogens and methods of using these compositions for the treatment of gastric and duodenal ulcers and gastrin induced cancers. The present invention concerns the use of the same anti-G17 inmunogens to treat disease states such as gastrointestinal cancers which are affected by the prohormone G17-Gly.

In the present invention, a serum sample from the patient having a gastrointestinal cancer can be assayed to determine the level of G17-Gly in the patient's blood. An effective dosage ranging from 0.001 to 2mg of the immunogenic composition is administered to the patient for the treatment of the gastrointestinal cancer. The effective dosage of the immunogenic composition should be capable of eliciting an immune response in a patient of effective levels of antibody titer against both human gastrin 17 and the G17-Gly within 1-3 months after immunization. Following the immunization of a patient, the antibody titer levels against anidated or glycine-extended G17 are monitored from a sample of blood taken from the patient, and booster immunizations should be given as required to maintain an effective antibody titer which will neutralize G17-Gly and amidated G17. The effective antibody titer which will neutralize G17-Gly and amidated G17 is defined as the minimum antigen binding capacity of 5 picomoles of antigen-bound in one milliliter of the patient's serum, as measured by standard immunological assays. In addition, serum G17-Gly can be monitored to assess the effectiveness of immunization against G17. Effective treatment of gastrointestinal cancers such as colorectal adenocarcinomas, according to this method should result in inhibition of tumor growth and a decrease in size of the tumor.

The Antibody titers raised by the immunogens are in excess of those required to neutralize serum G17, resulting in high serum levels of uncomplexed antibodies which are free to bind to G17-Gly. Thus, the 'free' serum-associated antibodies would be available to neutralize cell-associated G17 peptides in veil-vascularized areas of the tumors.

Antibodies raised by then immunogens of used in the present invention may have significant anti-trophic effects against gastrointestinal cancer, such as a colon tumor by two potential mechanisms: (i) neutralization of serum G17, and (ii) neutralization of cell-associated G17-Gly molecules.

The following examples demonstrate the effect of active immunization with rat G17 immunogen on the in vivo growth of the rat colon cancer line, DHDK12. DHDK12 is a rat colonic tumor cell line of epithelial morphology (Martin et al. 1983). The immunogen tested is composed of the N-terminal 9 amino acids of G17 linked to DT by a spacer peptide, and can be made specific for either human or rat G17. Antiserum raised by anti-G17 immunization is denoted as anti-G17(1-9):DT and contains a spacer peptide.

### Example 1

These experiments demonstrate that the immunogen induces antisera that bind to amidated G17 and glycine-extended G17, but not to G34.

### Gastrin-specificity of antiserum raised by anti-G17 immunization of rabbits

Antisera were absorbed onto a solid phase at a concentration of 100µg/ml and displacement was determined in a competitive assay with a fixed concentration of radiolabelled G17 (1000 µg/ml) and increasing concentrations of unlabelled ligands (1-25,000 µg/ml).

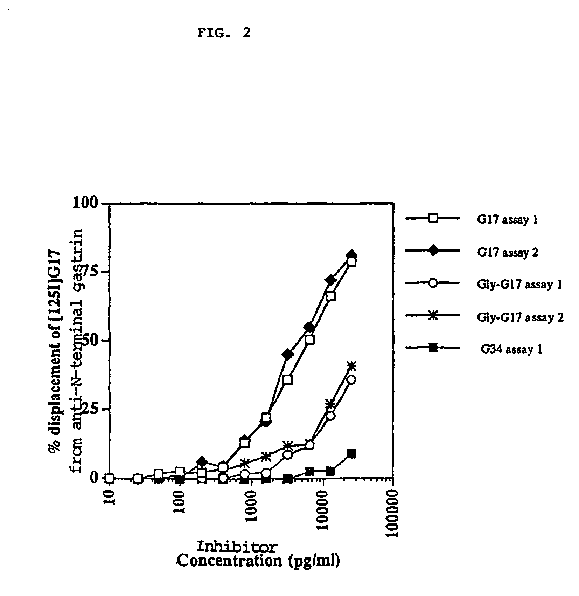

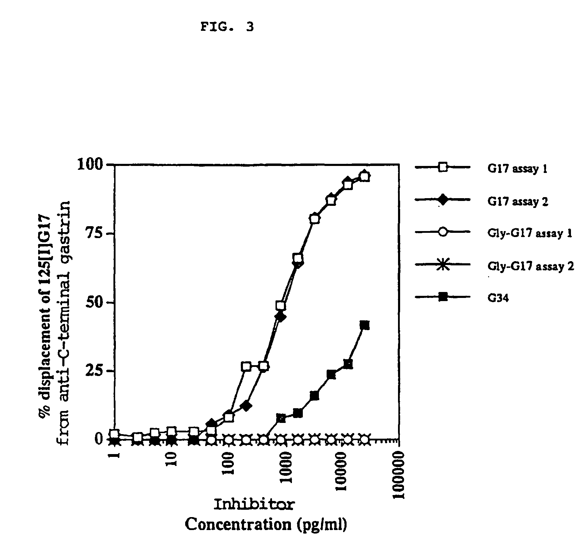

Figures 2 and 3 show the displacement of [¹²⁵I]G17 from rabbit anti-human G17 antiserum by G17, G17-Gly and G34. The antiserum used in the test depicted in Figure 2 was obtained from animals immunized with G17(1-9):DT and was specific for the N-terminal end of G17; the antiserum for Figure 3 was specific for the C-terminal end of G17. G17 displaced radiolabelled G17 from both antisera preparations with a 50% inhibitory concentration (IC₅₀) of 3500 pg/ml for the rabbit anti-human G17 (1-9):DT (N-terminal) and 800 pg/ml for the rabbit anti-G17 (C-terminal). Glycine-extended G17 did not displace radiolabelled G17 from the C-terminal specific antiserum, but did from the N-terminal specific antiserum (IC₂₅ 12,000 pg/ml), demonstrating that the glycine-extended G17 binds to N-terminal specific antiserum, but not to C-terminal specific antiserum. G34 displaced radiolabelled G17 from the C-terminal (IC₂₅ 500 pg/ml), but not the N-terminal specific antiserum, demonstrating the specificity of the G17(1-9):DT antiserum for G17 and glycine-extended G17 and not to G34.

### Example 2

These experiments show that the DHDK12 rat colonic cells produce glycine-extended gastrin 17 and that anti-G17 antiserum reduces the levels thereof produced by the cells.

### Radioimmune assay of glycine-extended and amidated gastrin levels

DHDK12 cells were grow to sub-confluence in RPMI 1640 culture medium (Gibco, Irvine, Scotland, UK) supplemented with 2mM glutamine (Sigma, Poole, Dorset, UK) and 10% heat-inactivated foetal calf serum (FCS, Sigma). The cells were incubated in humidified conditions at 37°C with 5% CO₂. cells were harvested with 0.025% EDTA (15 minutes at 37°C), washed by centrifugation and 2x10⁶ cells seeded into flasks containing serum-free medium (RPMI 1640 in a 1:1 ratio with Hams F12 (Gibco) with 0.5% bovine serum albumen [BSA]). Cells were harvested with 0.025% EDTA, washed, re-suspended in 1ml of sterile distilled water and heated in a boiling water bath. The levels of glycine-extended and amidated gastrin were measured by radioimmunoassay (RIA) using antibodies 109-21 and L-2, respectively, as described (Nemeth et al. 1993).

DHDK12 cells were shown to contain glycine-extended gastrin, but not amidated G17, in two separate experiments as shown in Table 1.

**Table 1 Glycine-extended and amidated gastrin levels associated with DHDK12 cells**

| | Glycine-extend ed G17 conc. | Amidated G17 conc (fmol/10⁷ cells) |
|---|---|---|
| | (fmol/10⁷ cells) | |
| Experiment 1 | 31.2 | ND¹ |
| (1.0 x 10⁷ cells/ml) | | |
| Experiment 2 | 80.0 | ND¹ |
| (1.27 x 10⁷ cells/ml) | | |

| | | |
|---|---|---|
| Tumor cell extracts were prepared by healing cells in 1ml of sterile water. Cell extracts were recovered by centrifugation and glycine-extended gastrin and amidated G17 levels were measured using antibodies 109-21 and L-2 respectively, as previously described (Nemeth et al. 1993). ¹ND - Not detected | | |

### Effect of rabbit anti-ratG17:DT treatment on the glycine-extended and amidated gastrin levels of DHDK12 cells

Semi-confluent DHDK12 cell monolayers were prepared as described previously in serum-free medium and harvested with 0.025% EDTA. Affinity/purified rabbit anti-ratG17:DT and rabbit anti-DT (negative control) were then added to the flasks at equivalent protein concentrations to give an antigen binding capacity for the former of 3 ng/ml. The cells were incubated for 4 days, after which cell extracts were prepared and assessed for glucine-extented and amidated, gastrin levels by were described above, with the results shown in Table 2.

**Table 2 Glycine-extended and amidated gastrin levels of DHDK12 cells after in vitro treatment with rabbit anti-G17(1-9):DT antiserum**

| **Treatment** | Glycine-extended G17 conc. | Amidated G17 conc (fmol/10⁷ cells) |
|---|---|---|
| | (fmol/10⁷ - cells) | |
| Rabbit anti-G17(1-9):DT antiserum | ND | ND |
| Rabbit anti-DT antiserum | 67.0 | ND¹ |

| | | |
|---|---|---|
| DHDK12 cells were grown in serum-free medium (RPMI 1640 in a 1:1 ratio with Hams F12 with 0.5% bovine serum albumen). Affinity purifies rabbit anti-rat G17(1-9):DT and rabbit anti-DT were then added to the flask at a protein concentration of 3 ng/ml and incubated for 4 days. Cell extracts were recovered by centrifugation and glycine-extended gastrin and amidated G17 levels were measured using antibodies 109-21 and L-2 respectively, as previously described (Nemeth et al. 1993). ¹ND Not detected | | |

As can be seen in Table 2, rabbit anti-ratG17:DT antiserum reduced the levels of glycine-extended G17 from 67 pg/ml to undetectable.

DHDK12 cells were also shown to express cell-associated glycine-extended G17 but not amidated gastrin. In vitro treatment of DHDK12 cells with rabbit anti-ratG17 (1-9): DT reduced the levels of cell-associated glycine-extended G17 when compared to cells treated with rabbit anti-DT control antiserum. Thus, antibodies produced by anti-G17 immunization may interrupt an autocrine growth loop involving such peptides as a consequence of down-regulation of gastrin translation.

### Example 3

The following experiments demonstrate that immunization of rats with the Rat G-17 (1-9) DT immunogen markedly inhibits then growth of DHDK12 tumors in vivo.

### Experimental animals

Male BDIX rats (The Animal Units, university of Liverpool, UK) of age 6-20 weeks weighing 340-430g were housed in pairs and maintained in a cycle of 12 hours light and 12 hours dark at 25°C with 50% humidity. The rats were allowed to acolimatize for at least 7 days before use.

### Immunization procedure

Rat G17(1-9) coupled to DT or the DT component alone were dissolved in sterile saline (0.9%), pH 7.3 at 1mg/ml. The adjuvant nor-muranyl dipeptide (nor-MDP, Peninsula Labs. CA) was added to the conjugate to give a final concentration of 500 µg/ml. The aqueous solution was mixed with oil (Montanide ISA 703 AMS Seppic, Inc., Paris, France) in a 1:1 ratio (vol:vol) and placed in a glass syringe which was attached to a second syringe with a three-way stopcock as connector and the mixture forced back and forth through the syringes 100 times (the stopcock produced a right angle shear to assist emulsification).

Control animals received an identical emulsion containing the DT peptide only, and all experimental groups were equalized with respect to weight. A 200 µl volume of the emulsion was injected subcutaneously (s.c.) in the right hand flank of the experimental animal. The animals were immunized at 21 day intervals and the tumor implanted after 5 immunizations.

### Initiation of tumor growth

DHDK12 cells were suspended in sterile 0.9% saline at a concentration of 2.5x10⁷/ml. Rats were anaesthetized by a 1ml injection of Hypnorm (Fentanyl citrate 0.315 ng/ml and Fluanisone 10 mg/ml, Jannsen, Belgium), Hypnovel (Midazolan 5 ng/ml, Roche, Switzerland) and sterile distilled water in a 1:1:5 ratio. Following a s.c. incision on the right flank, a 200 µl volume of the cell suspension was injected into the muscle layer of the abdominal wall and the surgical incision closed with a wound clip. Each experimental group consisted of between 16-18 rats.

### Effect of rat Anti-G₁₇ immunization on the in vivo growth of DHDK12 tumors

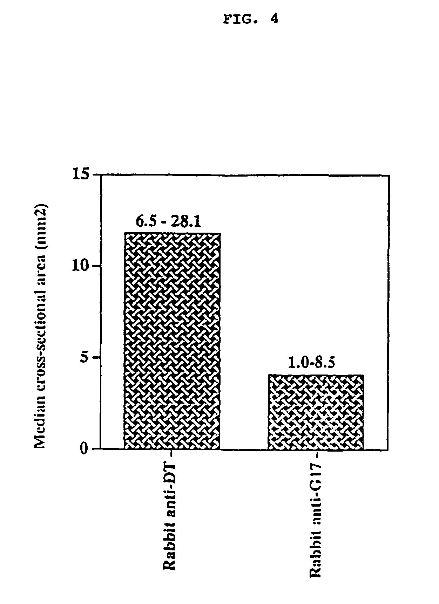

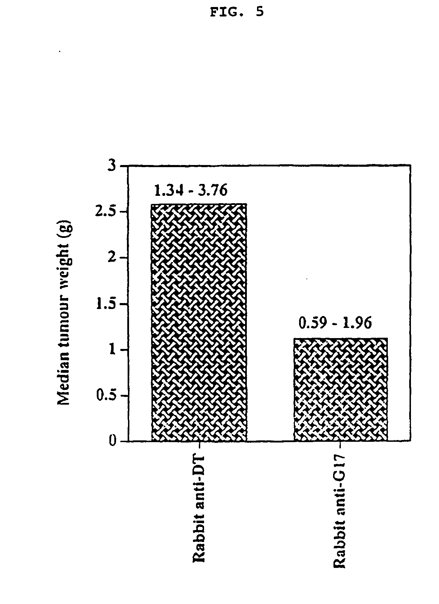

Figures 4 and 5 show the effect of immunization with rat G17(1-9)-DT immunogen (5 immunizations prior to injection of cells) on the final cross-sectional areas and weights, respectively, of DHDK12 tumors. The tumors had significantly reduced cross-sectional areas in rats immunized with anti-G17 immunogen. Figure 4 illustrates data which show that the median cross-sectional areas of tumors from anti-G17 treated rats were reduced by 70.2% when compared to tumors from the DT controls, p=0.005, Mann Whitney. DHDK12 tumors also had significantly reduced tumor weights in rats immunized with anti-G17 immunogen. Figure 5 shows that DHDK12 tumor weights were reduced by 56.5% when compared to tumors from the DT controls, p=0.0078. The mean animal weight in the anti-G17 treated rats rose from 399 g to 452 g (13% increase) over the duration of the experiment and in the DT- treated animals from 392 g to 447 g (13.8% increase) indicating that the growth rate of the animals was not affected by administration of the G17(1-9)-DT immunogen.

### Example 4

The experiments show the levels of anti-rat G17 antibodies induced in immunized rats that were implanted with DHDK12 tumors.

### Anti-rat G17 antibody levels of rat G17 (1-9): DT-immunised rats

To determine the antibody response to the emulsified rat G-17(1-9)DT immunogen, rats were tail-bled at various time points and an ELISA technique was used to determine the anti-rat G17:DT antibody titers.

A rat G17-BSA conjugate was prepared at a concentration of 2 µg/ml in Glycine buffer (0.1M, pH 9.5) and 25 µl was plated per well into 96-well Immunolon U plates (Dynatech Labs., Sussex, UK) and incubated overnight at 4°C. The unabsorbed conjugate was then flicked out and the wells washed in buffer which consisted of 0.9% saline, pH 7.3 containing 0.5% Tween-20 (Sigma) and 0.02% NaN₃ (Sigma). This buffer was used for both washing and reagent dilutions. The test sera (from animals immunized with the rat gastrin immunogen) were used at a starting dilution of 1:100 and at 10 fold dilutions thereafter. The positive control was rat anti-rat G17 antiserum, from previously immunized animals and the negative controls were normal rat serum and sera from rats immunized with DT. These were used at the same dilutions as described for the test sera. The test and control sera were added to the wells in 25 µl volumes either in the absence or presence of 25 µl/well rat G17-BSA at 100 µg/ml (control wells received 25 µl assay buffer). The plates were then incubated for 60 minutes at room temperature. The plates were washed with saline buffer, then groat anti-rat Ig (H+L)-biotin (Zymed, San Francisco, CA) was added to the wells at a 1:500 dilution, 50 µl/well, and incubated for 60 minutes in the dark at room temperature. The plates were washed with saline buffer and avidin alkaline phosphatase (Zymed) was added to wells at a 1:100 dilution, 50 *µ*l/well and incubated for 60 minutes in the dark at room temperature. After washing with saline buffer, p-nitro-phenylphosphate (pNPP) substrate (Sigma) in substrate buffer was added to the wells at 50 µl/well and after 5 minutes developing time the absorbance was read at 405nm. The difference in absorbance between untreated sera and sera co-incubated with rat G17-BSA was calculated as the specific absorbance.

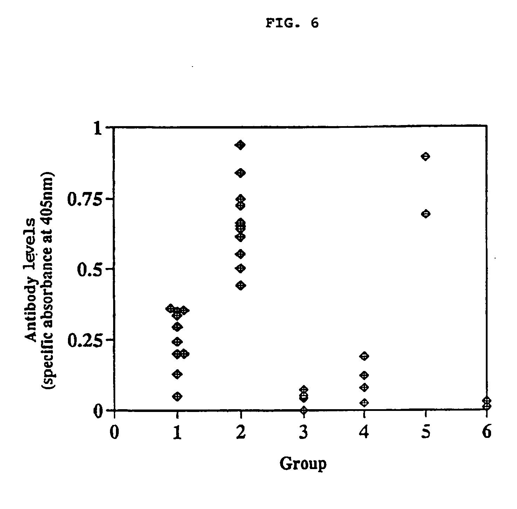

The free anti-rat G17(1-9):DT antibody levels (those in excess of the antibodies required to bind serum-associated G17) were measured and are expressed as the specific absorbance obtained at a 1:100 dilution of serum (Figure 6). After 5 immunizations, at the time of tumor cell injection, the mean antibody level was 0.243 absorbance units (Group 1 in Figure 6). The mean antibody level had increased, by the termination of the study, following 2 further immunizations, to 0.66 absorbance units (Group 2 in Figure 6) and were in the range of the positive control (Group 3 in Figure 6). Antibody levels from animals immunized with DT had a mean absorbance of 0.1 units (Groups 4 and 5 in Figure 6) and the negative control (normal rat serum) showed no absorbance (Group 6 in Figure 6). There was no apparent correlation between tumor weight and antibody levels as measured by a Linear Regression Analysis, (p=0.14).

### Example 5

The following experiments show that immunization against G17 reduced serum G17 levels and that these reduced levels correlated with reduced tumor growth.

### G17 levels in the immunized rats as determined by an inhibition RIA

Rabbit anti-G17 antiserum (C-terminal-specific, Dakopatts, Bucks., UK) was coated onto 96-well microtitre plates at a protein concentration of 10 ng/well in PBS. A standard curve was constructed by incubating [¹²⁵I]G17 at a fixed concentration of 10,000 CPM/well with increasing concentrations of G17.

The unknown samples containing free gastrin, bound G17, free and bound anti-G17 antibodies were prepared in 250 µl aliquots. A 125 µl aliquot of newborn calf serum (Sigma) and 312.5 µl of 25% polyethylene glycol (Sigma) were added to each sera sample. These were vortexed and spun at 1500 rpm for 30 minutes. The supernatant was removed and boiled (to ensure no free antibodies remained) and was classified as the free gastrin sample.

The pellet was washed 5 times in 0.002 M veronal buffer (pH 8.4) containing 0.5% bovine serum albumen and solubilized by boiling in 250 µl of water. This was classified as containing bound gastrin. Triplicate aliquots of each of the samples were added to the labelled G17 and the level of inhibition was determined. Gastrin levels in the rat sera were then calculated from the standard curve.

The free serum gastrin level (as measured with an antiserum directed against the carboxy terminus of G17) of the DT-immunized rats was found to be 114.0 pg/ml (standard deviation of 31) compared to 68.5 pg/ml (standard deviation of 20) in the rat G17(1-9): DT-immunized group. This corresponds to a 40% reduction in total gastrin. Total serum gastrin levels were correlated with final tumor weight and the correlation coefficient was found to be statistically significant, (p=0.011, Linear Regression Analysis. The levels of serum gastrin bound to antibodies was found to be zero in the DT-immunized rats and ranged from 30.1 to 253.7 pg/ml in the rat G17(1-9): DT-immunized rats (median of 53.3 pg/ml).

DHDK12 rat colon tumor cells growing in vivo, by virtue of their CCKB/gastrin receptors, have responded to serum G17. In these experiments, excess anti-G17 antibodies (i.e. those not bound to serum G17) were measured during the tumor challenge. The total serum gastrin levels were shown to be reduced by 40% and a significant positive correlation was shown between tumor weight and serum gastrin levels at the termination of the therapy. In addition, antibody-bound gastrin was also detected in the rat G17(1-9): DT immunized, but not in the DT-immunized, rats. Thus, neutralization of serum-associated gastrin contributed to reduced tumor growth.

### Example 6

The experiments show that immunization against G17 affects the histological appearance of DHDK12 tumors.

### Histological evaluation of the rat tumors

At termination of therapy, the DHDK12 tumors were fixed in lot formal calcium and embedded in paraffin. 5 µm sections were cut on a cryostat, stained with Haematoxylin and Eosin and the pathological parameters of the tumors assessed independently by a Pathologist. Image analysis was performed on the tumor sections using a Seescan Image Analyzer in a blind manner to assess the area of viable tumor tissue.

Gastrin receptors (GR) were detected using a rabbit anti-CCKB/gastrin receptor polyclonal antiserum. Sections were incubated with a 1:500 dilution overnight at 4°C. Binding was detected using the avidin-biotin technique with immunoperoxidase as the enzyme tracer and diaminobenzidine as the substrate.

Histological evaluation revealed that the tumors from rat G17(1-9): DT-immunized rats had a smaller rim of viable tumor tissue around the leading edge of the tumor and a greater degree of central necrosis when compared to tumors from rats immunized with DT. This was quantified by image analysis and the mean percentage of viable cell area in tumors from rat G17(1-9):DT-treated rats was 40.3% (standard deviation of 9.1) compared to 58.6% (standard deviation of 10.4) for the DT-immunized rats (p=0.003, student's t test).

Higher magnification microscopy showed that the tumor cells in the DT-immunized rats grew in a regular trabecular manner, whereas the tumor cells from G17(1-9):DT-immunized rats had a disrupted pattern of growth. There was also more connective tissue in the tumors from G17(1-9): DT-treated rats when compared to tumors from DT-treated rats (connective tissue:tumor ratio being 75:25 and 50:50, respectively). Areas of focal necrosis were present within the viable tumor tissue in the G17(1-9):DT treated group and also an increased inflammatory infiltrate which appeared to be composed mostly of lymphocytes. The tumors from rats in both treatment groups were stained with anti-GR antiserum and it was shown that the viable cells remaining in both the DT and the G17(1-9):DT treated groups had retained their GR positivity.

Immunization with the G17:DT immunogen reduces the in vivo growth of DHDK12 rat colon tumors as shown by both cross-sectional area and weight measurements. Extrapolation of quantitative assessment of viable tumor tissue by image analysis indicated that the weight of viable tumor tissue may have been reduced by as much as 68%.

One further finding was the focal areas of necrosis within the tumor tissue in anti-G17 treated rats and the presence of an inflammatory infiltrate in certain areas of the tumor which was mainly composed of lymphocytes. One possible explanation of such findings is that an antibody-dependent cellular cytotoxic response was instigated by the anti-G17 immunogen. The mechanism of such a response as applied to immunization against G17 is unknown.

Anti-G17 immunization resulted in the potential neutralization of two trophic forms of gastrin, G17 and glycine-extended G17, and thus may induce cytostasis within the tumors. The histological observations provide evidence for the theory that tumors from anti-G17 immunogen treated rats have a slower growth rate than tumors from control rats as the growth pattern, the degree of fibrosis and the area of the viable tumor tissue was significantly reduced in the former rats. Interestingly, the viable tumor cells remaining were shown to maintain their expression of GR. This indicates that in this tumor model, the gastrin hormone-sensitive phenotype may have been expressed by all the cell clones and there was no outgrowth of gastrin hormone-insensitive clones leading to escape from anti-G17 immunogenic inhibition.

### Example 7

### Immunocytochemical evaluation of the CCXB/gastrin receptor expression of DHDK12 cells

DHDK12 cells were suspended at a concentration of 1x10⁶/ml and 200 µl volumes were cytospun onto microscope slides (1200 rpm, 5 minutes). The cells were fixed with methanol at -20°C (5 minutes) and permeabilized by treatment with graded alcohols. The cells were incubated with the rabbit anti-CCKB/gastrin receptor antiserum and stained as previously described.

CCKB/gastrin receptor expression of DHDK12 cells was evaluated with antiserum raised against peptide sequences derived from the human CCKB/gastrin receptor. DHDK12 cells showed a strong and specific membrane-associated immunoreactivity indicative of a high level of gastrin receptor expression. Cells treated with a control rabbit antiserum showed no specific immunoreactivity.

### References

1 Watson, S.A., and Steele, R.J.C. Gastrin receptors in GI tumors. R.G. Landes, Austin, 1993.
2 Rehfeld, J.F. Three components of gastrin in human serum. Biochim. Biophys. Acta., 285: 364-372, 1972.
3 Upp, J.R., Singh, S., Townsend, C.M., and Thompson, J.C. Clinical significance of gastrin receptors in human colon cancers. Cancer Res., 49: 488-492, 1989.
4 Hoosein, N.M., Kiener, P.A., Curry, R.C., and Brattain, M.G. Evidence for autocrine growth stimulation of cultured colon tumor cells by a gastrin/cholecystokinin-like peptide. Exptl. Cell Res., 186: 15-21, 1990.
5 Baldwin, G.S., and Zhang, Q-X. Measurement of gastrin and transforming growth factor a messenger RNA levels in colonic carcinoma cell lines by quantitative polymerase chain reaction. Cancer Res., 52: 2261-2267, 1992.
6 Finley, G.G., Koski, R.A., Melham, M.F., Pipas, J.M., and Meisler, A.I. Expression of the gastrin gene in the normal human colon and colorectal adenocarcinoma. Cancer Res., 53: 2919-2926, 1993.
7 Watson, S.A., Durrant, L.G., Wencyk, P.M., Watson, A.L., and Morris, D.L. Intracellular gastrin in human gastrointestinal tumor cells. J.N.C.I., 83: 866-872, 1991.
8 Hoosein, N.M., Kiener, P.A., and Curry, R.C. Anti-proliferative effects of gastrin receptor antagonists and antibodies to gastrin on human colon carcinoma cell lines. Cancer Res., 48: 7179-7183, 1988.
9 Van-Solingo, W.W., Nielsen, P.C., Friis-Hansen, L., Falkmer, U.G., and Rehfeld, J.F. Expression but incomplete maturation of progastrin in colorectal carcinomas. Gastroenterology, 104:1099-1107, 1993.
10 Nemeth, J., Taylor, B., Pauwels, S., Varro A., and Dockray, G.J. Identification of progastrin derived peptides in colorectal carcinoma extracts. Gut., 341 90-95, 1993.
11 Seva, C., Dickinson, C.J., and Yamada, T. Growth-promoting effects of glycine-extended progastrin. Science, 265: 410-412, 1994.
12 Bock, M.G., DiPardio, R.M., Evans, B.E., Rittle, K.E., Whitter, A., Veber, D, Anderson, E., and Freidinger, A. Benzodiazepine, gastrin and brain cholecystokinin receptor ligands: L-365,260. J. Med. Chem., 32: 13-17, 1989.
13 Hughes, J., Boden, P., Costall, B., Domeney, A., Kelly, E., Horwell, D.C., Hunter, J.C., Pinnock, R.D., and Woodruff, G.N. Development of a class of selective cholecystokinin type B receptor antagonists having potent anxiolytic activity. Proc. Natl. Acad. Sci., 87: 6728-6732, 1990.
14 Watson, S.A., Durrant, L.G., Elston, P., and Morris, D.L. Inhibitory effects of the gastrin receptor antagonist (L-365,260) on gastrointestinal tumor cells. Cancer, 68: 1255-1260, 1991.
15 Romani, R., Howes, L.G., and Morris, D.L. Potent new family of gastrin receptor antagonists (GRAs) produces in vitro and in vivo inhibition of human colorectal cancer cell lines. Procs. AACR, 35: 397 (abstract), 1994.
16 Makishimi, R., Larkin, P., Michaeli, D., and Gaginella, T.S. Active immunization against gastrin-17 with an N-terminal derived immunogen inhibits gastric and duodenal lesions in rats. Gastroent., 106: A824, 1994.
17 Martin, F., Caignard, A., Jeannin, J-F., Leclerc, A., and Martin, M. Selection of trypsin of 2 sublines of rat colon cancer cells forming progressive or regressive tumors. Int. J. Cancer, 32: 623-627, 1983.
18 Ohning, G.V., Wong, H.C., and Walsh, J.H. Differential kinetics for immunoneutralization of circulating gastrin by gastrin monoclonal antibody and its Fab, fragment in rats. Peptides, 15: 417-423, 1994.
19 Dickinson, C.J. Relationship of gastrin processing to colon cancer. Gastroenterology, 109: 1384-1388, 1995.
20 Ciccotosto, G.D., McLeish, A., Hardy. K.J., and Shulkes, A. Expression, processing, and secretion of gastrin in patients with colorectal carcinoma. Gastroenterology, 109: 1142-1153, 1995.

## Claims

1. Use of an anti-gastrin-17 immunogen in the manufacture of a formulation for treating mammalian gastrointestinal tumors dependent on glycine-extended gastrin-17, wherein:
the immunogen comprises an amino-terminal peptide of gastrin-17 conjugated to an immunogenic carrier by a spacer peptide, wherein the amino-terminal peptide of gastrin-17 comprises amino acids 1 to 9 of gastric-17 wherein amino acids I to 9 are the sequence pGlu-Gly-Pro-Trp-Leu-Glu-Glu-Glu-Glu; and
the immunogen induces sufficient anti-gastrin 17 antibody titer to neutralize amidated gastrin-17 and glycine extended-gastrin-17.

2. The use of claim 1, wherein the immunogenic carrier is Diphtheria toxin.

3. Use of an anti-gastrin-17 antibody in the manufacture of a medicament for treating mammalian gastrointestinal tumors dependent on glycine-extended gastrin-17, wherein the antibody neutralizes amidated gastrin-17 and glycine extended-gastrin-17.

4. The use of any of claims 1-3, wherein the gastrointestinal tumor is a colorectal tumor.

5. An anti-gastrin-17 immunogen for use in treating mammalian gastrointestinal tumors dependent on glycine-extended gastrin-17, wherein:
the immunogen comprises an amino-terminal peptide of gastrin-17 conjugated to an immunogenic carrier by a spacer peptide, wherein the amino-terminal peptide of gastrin-17 comprises amino acids 1 to 9 of gastrin-17 wherein amino acids I to 9 are the sequence pGlu-Gly-Pro-Trp-Leu-Glu-Glu-Glu-Glu; and
the immunogen induces sufficient anti-gastrin 17 antibody titer to neutralize amidated gastrin-17 and glycine extended-gastrin-17.

6. The immunogen of claim 5, wherein the immunogenic carrier is Diphtheria toxin.

7. The immunogen of claim 5 or claim 6, wherein the gastrointestinal tumor is a colorectal tumor.

8. An anti-gastrin-17 antibody for use in treating mammalian gastrointestinal tumors dependent on glycine-extended gastrin-17, wherein the antibody neutralizes amidated gastrin-17 and glycine extended gastrin-17.

9. The antibody of claim 8, wherein the gastrointestinal tumor is a colorectal tumor.

## Patentansprüche

1. Verwendung eines Anti-Gastrin-17- Immunogens zur Herstellung einer Formulierung zur Behandlung von Gastrointerstinaltumoren von Säugern, die von Glycin-extendiertem Gastrin-17 abhängig sind, worin:
das Immunogen ein Amino-terminales Peptid von Gastrin-17, konjugiert an einen Immunogen-Träger durch ein Spacer-Peptid, umfasst, worin das aminoterminale Peptid des Gastrin-17 die Aminosäuren 1 bis 9 von Gastrin-17 umfasst, worin die Aminosäuren 1 bis 9 die Sequenz pGlu-Gly-Pro-Trp-Leu-Glu-Glu-Glu-Glu sind; und
worin das Immunogen ausreichend Anti-Gastrin-17-Antikörpertiter induziert, um amidiertes Gastrin-17 und Glycin-extendiertes Gastrin-17 zu neutralisieren.

2. Verwendung nach Anspruch 1, worin der Immunogen-Träger Diphterietoxin ist.

3. Verwendung eines Anti-Gastrin-17-Antikörpers bei der Herstellung eines Medikaments zur Behandlung von Gastrointestinaltumoren von Säugern, die von Glycin-extendiertem Gastrin-17 abhängig sind, worin der Antikörper amidiertes Gastrin-17 und Glycin-extendiertes Gastrin-17 neutralisiert.

4. Verwendung nach einem der Ansprüche 1-3, worin der Gastrointestinaltumor ein Kolorektaltumor ist.

5. Anti-Gastrin-17- Immunogen zur Verwendung bei der Behandlung von Gastrointerstinaltumoren von Säugern, die von Glycin-extendiertem Gastrin-17 abhängig sind, worin:
das Immunogen ein Amino-terminales Peptid von Gastrin-17, konjugiert an einen Immunogen-Träger durch ein Spacer-Peptid, umfasst, worin das aminoterminale Peptid des Gastrin-17 die Aminosäuren 1 bis 9 von Gastrin-17 umfasst, worin die Aminosäuren 1 bis 9 die Sequenz pGlu-Gly-Pro-Trp-Leu-Glu-Glu-Glu-Glu sind; und
worin das Immunogen ausreichend Anti-Gastrin-17-Antikörpertiter induziert, um amidiertes Gastrin-17 und Glycin-extendiertes Gastrin-17 zu neutralisieren.

6. Immunogen nach Anspruch 5, worin der Immunogen-Träger Diphterietoxin ist.

7. Immunogen nach Anspruch 5 oder Anspruch 6, worin der Gastrointestinaltumor ein Kolorektaltumor ist.

8. Anti-Gastrin-17-Antikörper zur Verwendung bei der Behandlung von von Gastrointerstinaltumoren von Säugern, die von Glycin-extendiertem Gastrin-17 abhängig sind, worin der Antikörper amidiertes Gastrin-17 und Glycin-extendiertes Gastrin-17 neutralisiert.

9. Antikörper nach Anspruch 8, worin der Gastrointestinaltumor ein Kolorektaltumor ist.

## Revendications

1. Utilisation d'un agent immunogène anti-gastrine-17 dans la production d'une formulation destinée au traitement chez des mammifères de tumeurs gastro-intestinales dépendant de la gastrine-17 étendue avec la glycine, dans laquelle :
l'agent immunogène comprend un peptide amino-terminal de gastrine-17 conjugué avec un support immunogène au moyen d'un peptide intercalaire, ledit peptide amino-terminal de gastrine-17 comprenant les aminoacides 1 à 9 de la gastrine-17, lesdits aminoacides 1 à 9 représentant la séquence pGlu-Gly-Pro-Trp-Leu-Glu-Glu-Glu-Glu ; et
l'agent immunogène induit un titre d'anticorps anti-gastrine-17 suffisant pour neutraliser la gastrine-17 amidée et la gastrine-17 étendue avec la glycine.

2. Utilisation suivant la revendication 1, dans laquelle le support immunogène est la toxine diphtérique.

3. Utilisation d'un anticorps anti-gastrine-17 dans la production d'un médicament destiné au traitement chez les mammifères de tumeurs gastro-intestinales dépendant de la gastrine-17 étendue avec la glycine, dans laquelle l'anticorps neutralise la gastrine-17 amidée et la gastrine-17 étendue avec la glycine.

4. Utilisation suivant l'une quelconque des revendications 1 à 3, dans laquelle la tumeur gastro-intestinale est une tumeur colorectale.

5. Agent immunogène anti-gastrine-17 destiné à être utilisé dans le traitement chez les mammifères de tumeurs gastro-intestinales dépendant de la gastrine-17 étendue avec la glycine, dans lequel :
l'agent immunogène comprend un peptide amino-terminal de gastrine-17 conjugué avec un support immunogène par un peptide intercalaire, ledit peptide amino-terminal de gastrine-17 comprenant les aminoacides 1 à 9 de la gastrine-17, lesdits aminoacides 1 à 9 représentant la séquence pGlu-Gly-Pro-Trp-Leu-Glu-Glu-Glu-Glu ; et
l'agent immunogène induit un titre d'anticorps anti-gastrine-17 suffisant pour neutraliser la gastrine-17 amidée et la gastrine-17 étendue avec la glycine.

6. Agent immunogène suivant la revendication 5, dans lequel le support immunogène est la toxine diphtérique.

7. Agent immunogène suivant la revendication 5 ou la revendication 6, dans lequel la tumeur gastro-intestinale est une tumeur colorectale.

8. Anticorps anti-gastrine-17 destiné à être utilisé dans le traitement chez les mammifères de tumeurs gastro-intestinales dépendant de la gastrine-17 étendue avec la glycine, ledit anticorps neutralisant la gastrine-17 amidée et la gastrine-17 étendue avec la glycine.

9. Anticorps suivant la revendication 8, dans lequel la tumeur gastro-intestinale est une tumeur colorectale.
